# EUROPEAN PATENT APPLICATION

(11) **EP 1 547 602 A1**
(43) Date of publication of application: **29.06.2005**
(21) Application number: 03799087.6
(22) Date of filing: 26.08.2003
(51) Int. Cl.: A61K 31/616, A61P 17/02, A61P 17/04

(54) **EXTERNAL PREPARATION FOR INHIBITING KELOID FORMATION**

(30) Priority: 30.09.2002 JP 2002285650
(71) Applicant: Teikoku Seiyaku Co., Ltd., Higashikagawa-shi, Kagawa 769-2695 (JP)
(72) Inventor: KAWAZOE, Takeshi, Kyoto-shi, Kyoto 606-8413 (JP); SUZUKI, Shigehiko, 1303, Forumu-higashinotoinsanjo, Nakagyo-ku, Kyoto-shi, Kyoto 604-813 (JP); INAMOTO, Yukiko, Kagawa-gun, Kagawa 761-1703 (JP); KAWADA, Mitsuhiro, Higashikagawa-shi, Kagawa 769-2702 (JP)
(74) Representative: Albrecht, Thomas, Dr.
(86) International application number: PCT/JP2003/010728
(87) International publication number: WO 2004/030676

(57) **Abstract**

An external preparation which contains acetylsalicylic acid or its salt as an active ingredient and has an effect of inhibiting keloid and/or hypertrophic scar formation as well as an analgesic and antipruritic effect.

## Description

### TECHNICAL FIELD

The present invention relates to an external preparation having an effect of inhibiting keloid and/or hypertrophic scar formation (cicatrization) as well as an analgesic and antipruritic effect on the said affected part.

In more detail, the present invention relates to an external preparation which contains acetylsalicylic acid or its pharmacological salt as an active ingredient and has an excellent effect of inhibiting keloid and / or hypertrophic scar formation as well as an analgesic and antipruritic effect on the said affected part, and the locally therapeutic system by using thereof.

### BACKGROUND ART

Until now, as therapies of keloid by drugs, the spread of steroids or the injection of steroids into the keloid lesion have been carried out. However, the former does not show any definite effect, and the latter requires several ten times injections repeatedly and gives the patient great pain on the injection though showing a certain effect. It is difficult to inject large amounts of the steroid once and therefore, the latter is hardly applied to large scaled keloid. There are such demerits on the known therapies.

On the other hand, the compression therapy such as directly pressing keloid by a sponge is also practiced. However, it takes long terms, several months to several years in this therapy.

Furthermore, keloid formed in the therapeutic course of wound by heating, etc., gives a great influence in the form of physical or mental sequelae to the patient and on his social activity after the therapy.

Keloid distresses the patient due to severe pruritus, strong pain, shrink-feeling, stiff-feeling, etc., peculiar to keloid as well as keloid makes worse the appearance. Therefore, if the keloid formation is protected in the course of the therapy of wound or dermal injury, the quality of life of the patient will be greatly improved.

However, in the course of the therapy of wound and dermal injury the external medicament having an effect of inhibiting keloid formation and/or hypertrophic scar formation without delaying the healing has not been approved and therefore, the medicament effective for inhibiting keloid formation and/or hypertrophic scar formation has been desired.

By the way, it is well-known that acetylsalicylic acid has been used as an antifebrile and an analgesic with safety and with a slight side effect.

It has not been reported that the inhibition of keloid formation and/or hypertrophic scar formation is confirmed by using the external medicament containing acetylsalicylic acid or its pharmacologically acceptable salt as an effective ingredient for the therapy of wound and dermal injury. In addition there is none of external medicaments having the analgesic and antipruritic effect on the lesion of keloid and/or hypertrophic scar together with the above inhibition effects on the keloid formation and/or hypertrophic scar formation, and it has not been disclosed that acetylsalicylic acid or its pharmacologically acceptable salt is effective for such conditions.

Keloid occurs due to temperature disturbance such as burn (ambustion), ulcer due to burn, frostbite, etc., external injuries such as fracture, abrasion, incised wound, morsus, acne, bite, etc., burger disease, blood vessel or limphotube injury such as limphedemas, crus ulcer, etc., postoperative wound such as donor site, sutural, etc., dermal wound such as decubitus, compressive ulcer, diabetic ulcer gangrene, stoma, radiation injury, chemical injury, etc. and dermal diseases such as dermal injury e.g., blister, erosion, etc. and the keloid is classified to ortho-keloid , scar keloid, hypertrophic scar, etc., depending on the course or condition.

### DISCLOSURE OF INVENTION

The present invention relates to solve the above problems and its object is to provide an external preparation having an excellent effect of inhibiting keloid and/or hypertrophic scar formation with a slight side effect as well as an analgesic and antipruritic effect on said affected part.

The present inventors have been extensively studied in solving the above problems and found that an external preparation which contains acetylsalicylic acid or its pharmacologically acceptable salt as an active ingredient has an excellent effect of inhibiting keloid and/or hypertrophic scar formation with a slight side effect and further, have a therapeutic effect on strong pain and pruritus peculiar to keloid.

Namely, when the external preparation containing acetylsalicylic acid as an active ingredient was applied to wound modeled animals or patients having keloid and/or hypertrophic scar, it was found as a result of the evaluation that the preparation showed the inhibition of shrinking (anastole) on the wound lesion after epithelialization without delaying wound healing, and that furthermore, showed the excellent inhibition effect to the subjective symptom such as pain or pruritus peculiar to keloid. It was found that the said external preparation was very useful as an agent for treating keloid and/or hypertrophic scar. Thus the present invention was completed.

### BEST MODE FOR CARRYING OUT THE INVENTION

The above activity and effect of the preparation depend on the amount of acetylsalicylic acid in the preparation, but the pharmacological activity is almost not influenced by more than the specific amount of the drug.

The present invention relates to an external medicament containing acetylsalicylic acid or its pharmacologically acceptable salt as an active ingredient having an effect of inhibiting keloid and/or hypertrophic scar formation, which preparation has analgesic and antipruritic activity together.

The present invention relates also to an external medicament containing acetylsalicylic acid or its pharmacologically acceptable salt as an active ingredient having analgesic and antipruritic activity on the lesion of keloid and/or hypertrophic scar.

The present invention relates to a method for inhibition of keloid and/or hypertrophic scar formation and for inhibition of pain or pruritus on the lesion of keloid and/or hypertrophic scar by administering a medicament containing acetylsalicylic acid or its pharmacologically acceptable salt in an effective amount to said lesion of a patient.

Furthermore, the present invention relates to a method for inhibition of pain or pruritus on the lesion of keloid and/or hypertrophic scar by administering a medicament containing acetylsalicylic acid or its pharmacologically acceptable salt in an effective amount to said lesion of a patient.

Acetylsalicylic acid used in the preparation of the present invention as an active ingredient is described in Japanese Pharmacopoeia, and the content of acetylsalicylic acid in said external preparation is different in accordance with the forms. The effective amount of the drug is 0.005∼80 % by weight per total weight, preferably 0.01~70 % by weight, more preferably 0.01~50 % by weight. When the amount of acetylsalicylic acid is less than 0.005 % by weight, the effect of acetylsalicylic acid is not fully shown and it is not preferable. When the amount of acetylsalicylic acid is beyond 80 % by weight, it becomes difficult to prepare the preparation.

The concentration of acetylsalicylic acid in the tissue which need the exhibition of inhibiting keloid and/or hypertrophic scar formation is 0.001~1500 µg/g per tissue weight, preferably 0.005~1000 µg/g per tissue weight, more preferably 0.01~800 µg/g per tissue weight.

The active ingredient contained in the external preparation of the present invention includes not only acetylsalicylic acid , but also its salt such as an amino acid salt, e.g., DL-lysine salt etc. or an organic salt e.g., sodium salt, etc.

The external preparation of the present invention is not limited as far as the preparation can be directly applicable to the surface of dermal lesion, such as ointments, creams, gels, ointment patches, solutions (suspensions, emulsions, lotions, etc.), cataplasms, tapes, external powders, aerosols, etc.

The ingredients used in the preparation of the present invention except acetylsalicylic acid are not limited as far as they are the ingredients usually used in the ordinarily external preparation.

The ointments, the creams, the gels and the lotions may contain a base such as white petrolatum, yellow petrolatum, lanolin, white beeswax, cetanol, stearyl alcohol, stearic acid, hydrogenated oil, hydrocarbon gel, polyethylene glycol, liquid paraffin, squalane, etc.; a solvent or a dissolving agent such as oleic acid, isopropyl myristate, glyceryl triisooctanoate, crotamiton, diethyl sebacate, disopropyl adipate, hexyl laurate, a fatty acid, a fatty acid ester, an aliphatic alcohol, a vegetable oil, etc.; an antioxidant such as a tocopherol derivative, L-ascorbic acid, dibutylhydroxytoluene, butylhydroxyanisole, etc.; an antiseptic such as parahydroxybenzoate, etc.; a humectant agent such as glycerin, propylene glycol, sodium hyaluronate, etc.; a surfactant such as a polyoxyethylene derivative, a glyceryl fatty acid ester, a sucrose fatty acid ester, a sorbitan fatty acid ester, a propylene glycol fatty acid ester, lecithin, etc.; a thickener such as carboxyvinylpolymer, xanthan gum, carboxymethylcellulose, sodium carboxymethylcellulose salt, hydroxypropyl cellulose, hydroxypropyl methylcellulose, etc.; a stabilizing agent; a preservative; an absorption promoting agent, etc.; or an suitable additive.

The cataplasms may contain a tackifier such as polyacrylic acid, polyacrylic acid copolymer, etc.; a crosslinking agent such as aluminum sulfate, aluminum potassium sulfate, aluminum chloride, magnesium alminometasilicate, dihydroxyalminum acetate, etc.; a thickener such as sodium polyacrylate, polyvinyl alcohol, polyvinylpyrrolidone, gelatin, sodium alginate, carboxymethylcellulose, carboxymethycellulose sodium salt, hydroxypropyl cellulose, hydroxypropyl methylcellulose, etc.; a polyvalent alcohol such as glycerin, polyethylene glycol (macrogol), propylene glycol, 1,3-butandiol, etc.; a surfactant such as polyoxyethylene derivative, etc.; a flavor such as 1-menthol, etc.; an antiseptic such as parahydroxybenzoate, etc.; purified water; or an suitable additive.

The tapes may contain an adhesive agent such as styreneisoprene-styrene block copolymer, acryl resin, etc., a tackifier resin such as alicyclic saturated hydrocarbon resin, rosin, terpene resin, etc.; a softener such as liquid rubber, liquid paraffin, etc.; an antioxidant such as dibutylhydroxytoluene, etc.; a polyvalent alcohol such as propylene glycol, etc.; an absorption promoting agent such as oleic acid, etc.; a surfactant such as a polyoxyethylene derivative, etc.; or an suitable additive. The aqueous tapes can be also prepared by blending an aqueous high molecular compound such as sodium polyacrylate or polyvinyl alcohol with a small amount of purified water.

The external powders may contain an excipient such as potato starch, rice starch, corn starch, talc, zinc oxide, etc. or a suitable additive.

The aerosols may contain the ingredients used in ointments, creams, gels, suspensions, emulsions, solutions, lotions and external powders, namely a base such as white petrolatum, yellow petrolatum, lanolin, white beeswax, cetanol, stearyl alcohol, stearic acid, dehydrogenated oil, hydrocarbon gel, polyethylene glycol, liquid paraffin, squalane, etc.; a solvent or a dissolving agent such as oleic acid, isopropyl myristate, diisopropyl adipate, isopropyl sevacate, glyceryl triisooctanoate, crotamiton, diethyl sevacate, hexyl laurate, a fatty acid, a fatty acid ester, an aliphatic alcohol, a vegetable oil, etc.; an antioxidant such as a tocopherol derivative, L-ascorbic acid, dibutylhydroxytoluene, butylhydroxyanisole, etc.; an antiseptic such as parahydroxybenzoate, etc.; a humidity preserving agent such as glycerin, propylene glycol, sodium hyaluronate etc.; a surfactant such as a polyoxyethylene derivative, a polyoxyethylene derivative, a glycerin fatty acid ester, a sucrose fatty acid ester, a sorbitan fatty acid ester, a propylene glycol fatty acid ester, lecithin, etc.; a stabilizer like a thickener such as carboxyvinyl polymer, xanthan gum, carboxymethylcellulose, sodium carboxymethylcellulose, hydroxypropyl cellulose, hydroxypropyl methylcellulose, etc.; an excipient such as potato starch, rice starch, corn starch, talk, zinc oxide, etc.; a jet such as liquid petroleum gas, liquid carbondioxide, dimethy ether, nitrogen, kerosine, carbon dioxide, etc.; a buffer; a sweetening agent; a suspending agent; an emulsifying agent; a flavor; a preservative; a solubilizing agent; or an suitable additive.

The external preparation of the present invention is prepared by well kneading each ingredient and, if necessary a base in accordance with the conventional method, and the preparation is practiced by directly applying it to the wound lesion or by spreading it on the cloth or immersing it to the cloth and then applying it in accordance with the usual application method.

The ointments is prepared by using fat, fatty oil, lanolin, wax, resin, plastic, glycol, a high molecular alcohol, glycerin, water, an emulsifying agent, a suspending agent or other suitable excipient as a starting material and kneading it with an active ingredient, or by using these ingredients as base ingredients and homogenously kneading them with an active ingredient. The base ingredients are melted under heating and stirred homogenously and if necessary, adding an additive such as an absorption promoting agent, an antioxidant, a preservative, a surfactant, purified water, etc. and further to the mixture was added fine powders of acetylsalicylic under stirring to give ointments or creams.

For example, oleaginous ointments are prepared by melting the base materials under warming and mixed well, followed by mild cooling. Then the drug which is melted or finely pulverized is mixed with a part of the base, and thereto is added the residual base. The mixture is homogenously kneaded to give oleaginous ointments.

For example, emulsion-type ointments or aqueous ointments are prepared as follows: The solid base is melted on a water bath, and kept at about 75°C. Thereto is added a solution prepared by dissolving an aqueous base in water which is warmed to the same temperature or a little higher, and the mixture is homogonously kneaded. When other drug is added thereto, in accordance with a kind of bases, the aqueous or oil-soluble drug is mixed with a part of the base, and then the residual base is added thereto and the mixture is homogenously kneaded.

The cataplasms are prepared as follows: The drug is previously kneaded with an ointment base containing mainly an aqueous high molecular weight which is rich in water preservability such as gelatin, calmellose sodium, methylcellulose, sodium polyacrylate, etc. and the mixture is spread on a backing such as a woven textile. The ointment surface is covered with a plastic film such as polyethylene or polypropylene, etc. and is cut in a desired size.

The tapes are prepared as follows: To an adhesive agent such as acrylic resin, etc. or styrene-isoprene-styrene block copolymer are added a tackifier resin such as an aliphatic saturated hydrocarbon resin, rosin, terpene resin, etc., an softener such as liquid rubber, liquid paraffin, etc., an absorption promoting agent, and an antioxidant, etc. and the mixture is dissolved in an organic solvent such as toluene, etc. or melted by heating, followed by stirring. Then the drug in a solution or in powders is added thereto, and the mixture is blended, spread on a release paper and in case of a soluble type, spread on a release paper and dried. It is laminated with a flexible backing such as a polyurethane film, a polyethylene film, a polyvinyl chloride film, woven fabrics, unwoven textile, etc. and is cut in a desired size.

The lotions are prepared as follows: The drug and a solvent, an emulsifying agent or a suspending agent are added to an aqueous solution to prepare homogenous solutions. Suspension type-lotions are prepared by finely pulverizing the drug, wetting it with glycerin or ethanol and then thereto gradually adding a suspending agent or a base for lotions. The mixture is homogenously mixed to prepare suspension-lotions. In addition, emulsion type-lotions are prepared as follows: The oil-soluble drug and an oil phase are poured in a vessel, and an aqueous phase is poured in another vessel. Both vessels are warmed. In case of preparing O/W type-emulsions, an oil phase is gradually added in an aqueous phase, and in case of preparing W/O type-emulsions, on the contrary an aqueous phase is added in an oil phase, and the mixture continues stirring to prepare homogenous solutions.

The external powders are prepared by homogenously dispersing acetylsalicylic acid and additives into excipients such as potato starch, rice starch, corn starch, talc, zinc oxide, etc.

The aerosols are prepared by preparing the drug-contained solutions, ointments, creams, gels, suspension, emulsion, solutions, lotions or external powders in the manner as mentioned above, and then putting them with a liquid gas or a pressured gas in a sealed vessel.

The dosage of the external preparation having an effect of inhibiting keloid and/or hypertrophic scar formation is not specially limited and is adequately chose in accordance with an administration mode, age of patients, the condition of diseases, an frequency of the disease and body weight of the patients.

The external preparation containing acetylsalicylic acid of the present invention is explained by Examples and Tests below, but the present invention should not be limited by them.

### Example

### Examples 1~7 (ointments)

According to the ingredients shown in Table 1, a base and a solvent were blended, and thereto was added acetylsalicylic acid. The mixture was well kneaded under stirring to give ointments.

**Table 1**

| Ingredients of ointment containing acetylsalicylic acid | | | | | | | |
|---|---|---|---|---|---|---|---|
| Example | 1 | 2 | 3 | 4 | 5 | 6 | 7 |
| Ingredient | % by weight | | | | | | |
| Acetylsalicylic acid | 0.05 | 0.25 | 2.0 | 4.0 | 10.0 | 5.0 | 5.0 |
| Sesame oil | | | | | | 5.0 | |
| Disopropyl adipate | 5.0 | 5.0 | 5.0 | 5.0 | 5.0 | | 5.0 |
| Isopropyl myristate | | | | | | | 5.0 |
| White petrolatum | | | | | | 90.0 | 90.0 |
| Hydrocarbon gel | 94.95 | 94.75 | 93.0 | 91.0 | 85.0 | | |

### Example 8 (solutions)

According to the ingredients shown in Table 2, acetylsalicylic acid was dissolved or dispersed in a solvent, and the mixture was vigorously added under stirring to warmed purified water in which other ingredients were dissolved, and the mixture continued stirring to become a completely homogenous liquid to give solutions.

**Table 2**

| Ingredients of solution containing acetylsalicylic acid | |
|---|---|
| Example | 8 |
| Ingredient | % by weight |
| Acetylsalicylic acid | 0.5 |
| Crotamiton | 1.0 |
| Squalane | 3.0 |
| Cetanol | 3.0 |
| Sorbitan sesquioleate | 0.5 |
| Polyoxy (20) cetyl ether | 1.5 |
| Propylene glycol | 5.0 |
| Triethanolamine | 0.4 |
| Purified water | 85.1 |

### Examples 9 and 10 (gels)

According to ingredients shown in Table 3, after an aqueous high molecular compound was dissolved by warming, acetylsalicylic acid was dispersed or dissolved in a solvent, and the mixture was kneaded with a residual base to become homogenous to give gels.

**Table 3**

| Ingredients of gel containing acetylsalicylic acid | | |
|---|---|---|
| Example | 9 | 10 |
| Ingredient | % by weight | |
| Acetylsalicylic acid | 0.1 | 5.0 |
| Crotamiton | 5.0 | 3.0 |
| Isopropanol | | 3.0 |
| Propylene glycol | 45.0 | 45.0 |
| Polyacrylic acid | 25.0 | 25.0 |
| Triethanolamine | 0.7 | 0.7 |
| Purified water | 24.2 | 8.3 |

### Example 11 (creams)

According to the ingredients shown in Table 4, after an oil base was melted on a water bath, acetylsalicylic acid which was dispersed or dissolved in the solvent was mixed therein. Warmed water in which an aqueous base was dissolved was added thereto and the mixture was kneaded to become homogenous to give creams.

**Table 4**

| Ingredients of cream containing acetylsalicylic acid | |
|---|---|
| Example | 11 |
| Ingredient | % by weight |
| Acetylsalicylic acid | 1.0 |
| Sesame oil | 5.0 |
| Cetanol | 9.0 |
| White petrolatum | 8.0 |
| Hexyldecanol | 1.0 |
| Polyethylene glycol monostearate | 2.0 |
| Polyoxy (9) lauryl ether | 2.8 |
| Polyoxy (23) cetyl ether | 2.0 |
| Propylene glycol | 12.0 |
| Methylparaben | 0.1 |
| Propylparaben | 0.1 |
| purified water | 57.0 |

### Example 12 (tapes)

According to the ingredients shown in Table 5, to an adhesive agent were added a thickener, a softener, an solvent, an absorption promoting agent, an antioxidant, etc. and the mixture was dissolved in an organic solvent such as toluene. The mixture was blended under stirring or melted under heating, and thereto was added acetylsalicylic acid, followed by stirring. The mixture was spread on a release paper and in case of a soluble type, spread and dried. The mixture was laminated with a flexible backing and cut in a desired size to give tapes.

**Table 5**

| Ingredients of tape containing acetylsalicylic acid | |
|---|---|
| Example | 12 |
| Ingredient | % by weight |
| Acetylsalicylic acid | 20.0 |
| Disopropyl adipate | 5.0 |
| Crotamiton | 1.0 |
| Styrene-isoprene-stylene block copolymer | 16.7 |
| Alicyclic saturated hydrocarbon resin | 32.8 |
| Polybutene | 13.3 |
| Liquid paraffin | 10.2 |
| Dibutylhydroxytoluene | 1.0 |

### Comparative examples 1 and 2

Comparative examples 1 and 2 had the ingredients respectively shown in Table 6.

**Table 6**

| Ingredients of ointment of Comparative example | | |
|---|---|---|
| Comparative example | 1 | 2 |
| Ingredient | % by weight | |
| Triamcinolone acetonide | 0.1 | |
| Salicylic acid | | 2.0 |
| Ethanol | 1.0 | 1.0 |
| White petrolatum | 99.4 | 98.8 |

### Comparative examples 3 and 4

Comparative examples 3 and 4 had the ingredients respectively shown in Table 7.

**Table 7**

| Ingredients of ointment of Comparative examples 3 and 4 | | |
|---|---|---|
| Comparative example | 3 | 4 |
| Marketed drug | A | B |
| Active ingredient (% by weight) | Dimethylisopropylazulene (0.03) | Bucladesine sodium (3.0) |

### Tests

The inhibition test of keloid and/or hypertrophic scar formation was carried out by the administration method to burn wound modeled animals, by collagen gel shrink inhibition test in vitro, and by administering to the lesion of keloid/hypertrophic scar of the patient (volunteers), followed by confirming on analgesic and antipruritic effects.

### Test 1

### Administration test to heat wound model by using type 2 diabetic modeled mice

Hairs on the back of type 2 diabetic modeled mice (male; 10 weeks old; Sugar value in blood 400-800 mg/dL; n=3) were cut and then, a heated iron (15mm × 15mm, 100°C, 5 sec) was touched on the skin with no pressure to make burn wound modeled mice. Thereafter each drug was applied once, and 3 weeks after application the wound lesion was macroscopically and histologically observed and evaluated. The result was shown in Table 8.

**Table 8**

| the change of wound lesion after epithelialization of heat wound modeled mice | | |
|---|---|---|
| Group | Acetylsalicylic acid | Area (mm²) |
| Ointment base | 0 | 84.0 |
| Example 2 | 0.25 | 126.7 |
| Example 3 | 2.0 | 150.1 |
| Example 4 | 4.0 | 177.1 |

Judging from the result of Table 8, the excellent effect of prevention of the scar contracture on the burn wound after epithelialization was confirmed in the groups of Examples 2, 3 and 4 containing acetylsalicylic acid, comparing with the group of ointment base-administration.

### Test 2

### Test of inhibition of collagen gel shrinking

A collagen solution (1.5 mg/mL) (1 mL) and a suspension of human normal fibroblast or human fibroblast derived from keloid (1×10⁵ cells) (1 mL) were added to a Petri dish (diameter; 35mm) and mixed. The mixture was warmed for about 10 minutes in an incubator (37°C, 5%CO₂) to prepare collagen gel. Further broth (1 mL) was added over thereon and was left for about 3 hours in the incubator (37°C, 5%CO₂). The collagen gel was released from the Petri dish, and thereto was added a solution containing acetylsalicylic acid (its final concentration was respectively 0, 0.05, 0.5, 5, and 50 µM). The suspension was cultured in the incubator (37°C, 5%CO₂) and after 24 hours the shrinking rate was evaluated.

The results were shown in Tables 9 and 10.

**Table 9**

| Shrinking rate of collagen (human normal fibroblast) | |
|---|---|
| Acetylsalicylic acid (µM) | Shrinking rate (%) |
| 0 | 26 |
| 0.05 | 23 |
| 0.5 | 12 |
| 5 | 10 |
| 50 | 14 |

**Table 10**

| Acetylsalicylic acid (µM) | Shrinking rate (%) |
|---|---|
| 0 | 30 |
| 0.05 | 21 |
| 0.5 | 19 |
| 5 | 24 |
| 50 | 20 |

As shown in Tables 9 and 10, the shrinking of collagen was inhibited in the group containing acetylsalicylic acid.

### Test 3

### Administration test to heat wound modeled rats

After hairs on the back of Wister male rats (10 weeks old; n=6) were cut, a heated iron (diameter: 12mm, 200°C, 5 sec) was touched on the skin to make heat wound modeled rats. The drug was administered to the wound lesion in 0.2 g/wound lesion once a day, and the days taking for healing were counted.

The result was shown in Table 11.

**Table 11**

| Days taking for healing in burn wound modeled rat | | | |
|---|---|---|---|
| Group | Drug (% by weight) | | Days for healing (day) |
| Untreated | - | | 28.5 |
| Ointment base | - | | 29.3 |
| Example 7 | Acetylsalicylic acid | 5.0 | 23.6 |
| Example 8 | Acetylsalicylic acid | 0.5 | 23.1 |
| Example 11 | Acetylsalicylic acid | 1.0 | 23.5 |
| Comparative example 2 | Salicylic acid | 2.0 | 29.6 |
| Comparative example 3 | Dimethyisopropyl azulene | 0.03 | 24.8 |
| Comparative example 4 | Bucladesine sodium | 3.0 | 23.9 |

As shown in Table 11, the delay of healing burn wound was not observed in the groups of Examples 7, 8 and 11 containing acetylsalicylic acid.

### Test 4

### Administration test to incised wound modeled rats

After hairs on the back of Wistar male rats (7 weeks old; n=15) were cut, all layer-incised wound was made and sutured. The drug was applied to the incised area of the incised wound modeled rats in 0.2g once a day for 7 days. Seven days after preparing the incised wound modeled rats, tension to be required to separate the skin peace on the incised wound area was measured and evaluated tension by a tensile compression test machine (Imada Company).

The result was shown in Table 12.

**Table 12**

| Tension on incised wound modeled rat | | | |
|---|---|---|---|
| Group | Drug (% by weight) | | Tension (kgf) |
| Untreated | - | | 1.01 |
| Ointment base | - | | 1.05 |
| Example 1 | Acetylsalicylic acid | 0.05 | 1.15 |
| Example 6 | Acetylsalicylic acid | 5.0 | 1.29 |
| Example 9 | Acetylsalicylic acid | 0.1 | 1.22 |
| Comparative example 4 | Bucladesine sodium | 3.0 | 1.23 |

As shown in Table 12, the tension in the groups of Examples 1, 6 and 9 containing acetylsalicylic acid was comparative to the tension of the group of Comparative example 4 of the commercialized drug for treating of wound and therefore, it was supposed that the former groups did not delay the wound healing.

### Test 5

### Improvement degree on pain or pruritus due to keloid

To patients suffering from keloid with pain or pruritus (total 19 patients), the external preparation containing acetylsalicylic acid was administered to the lesion, and the degree of improvement was evaluated.

The degree of improvement on pain or pruritus was evaluated on the following 5 stages; A: markedly effect, B: effective, C: slightly effective, D: no change, E: worse. When the improvement was more than slightly effective, the evaluation was judged effective, and the effective rate was calculated.

The result was shown in Table 13.

**Table 13**

| Improvement degree on pain or pruritus on patients suffering from keloid (burn) | | | | | | | | |
|---|---|---|---|---|---|---|---|---|
| Group | Drug (% by weight) | Patient (person) | Evaluation | | | | | Effective rate (%) |
| | | | A | B | C | D | E | |
| Ointment base | - | 2 | 0 | 0 | 0 | 1 | 1 | 0 |
| Example 3 | Acetylsalicylic acid | 3 | 0 | 2 | 1 | 0 | 0 | 100 |
| Example 5 | Acetylsalicylic acid | 4 | 0 | 2 | 1 | 1 | 0 | 75 |
| Example 10 | Acetylsalicylic acid | 3 | 1 | 1 | 1 | 0 | 0 | 100 |
| Example 12 | Acetylsalicylic acid 20.0 | 3 | 1 | 1 | 0 | 1 | 0 | 67 |
| Comparative example 1 | Triamcinolone 0.1 | 4 | 0 | 1 | 1 | 2 | 0 | 50 |

As shown in Table 13, the effect of more inhibition of pain or pruritus on patients suffering from keloid was confirmed in the groups Example 3, 5, 10 and 12 containing acetylsalicylic acid comparing with the groups of an ointment base and Comparative example 1.

### INDUSTRIAL APPLICABILITY

According to the present invention, an external medicament for inhibition of keloid and/or hypertrophic scar formation and an analgesic and antipruritic agent on the keloid and/or hypertrophic scar lesion and the method thereof can be provided by preparing the preparation containing acetylsalicylic acid or its pharmacologically acceptable salt as an active ingredient.

## Claims

1. An external medicament containing acetylsalicylic acid or its pharmacologically acceptable salt as an active ingredient, for inhibition of keloid and/or hypertrophic scar formation, which has analgesic and antipruritic activity together.

2. An external medicament containing acetylsalicylic acid or its pharmacologically acceptable salt as an active ingredient for analgesic and antipruritic on the lesion of keloid and/or hypertrophic scar.

3. A method for inhibition of keloid and/or hypertrophic scar formation and for inhibition of pain or pruritus on the lesion of keloid and/or hypertrophic scar by administering a medicament containing acetylsalicylic acid or its pharmacologically acceptable salt in an effective amount to said lesion of a patient.

4. A method for inhibition of pain or pruritus on the lesion of keloid and/or hypertrophic scar by administering a medicament containing acetylsalicylic acid or its pharmacologically acceptable salt in an effective amount to said lesion of a patient.
